# EUROPÄISCHE PATENTANMELDUNG

(11) **EP 3 254 705 A1**
(43) Veröffentlichungstag der Anmeldung: **13.12.2017**
(21) Anmeldenummer: 17173801.6
(22) Anmeldetag: 31.05.2017
(51) Int. Cl.: A61L 2/26

(54) **BEHANDLUNGSKAMMER IN LEICHTBAUWEISE**

(30) Priorität: 08.06.2016 DE 102016110572
(71) Anmelder: B. Braun Melsungen AG, 34212 Melsungen (DE)
(72) Erfinder: GEIGER, Ralph, 34587 Felsberg (DE); GUNDLACH, Christian, 34277 Fuldabrück (DE)
(74) Vertreter: Winter, Brandl, Fürniss, Hübner, Röss, Kaiser, Polte - Partnerschaft mbB

(57) **Zusammenfassung**

Die vorliegende Erfindung betrifft eine Behandlungskammer (1) zur Behandlung von Artikeln, insbesondere von medizintechnischen Artikeln, Geräten und Einrichtungen, bevorzugt im Rahmen von Sterilisationsprozessen, mit einem Behandlungsraum, der durch ein unteres Wandelement (2), eine Mehrzahl von seitlichen Wandelementen (3, 4, 6) und ein oberes Wandelement (5) umgeben ist, wobei der Behandlungsraum zur Erzeugung eines gewünschten Innendrucks darin hermetisch dicht oder abdichtbar und zur Erzeugung einer gewünschten Innentemperatur darin temperierbar ist, wobei zumindest eines der Wandelemente (2, 3, 4, 5, 6) aus zumindest einem Hohlkammerprofil (8, 9) ausgebildet ist.

## Beschreibung

Die vorliegende Erfindung betrifft eine Behandlungskammer, insbesondere eine medizintechnische Behandlungskammer, zur Behandlung von Artikeln, bevorzugt von medizintechnischen, Artikeln, Instrumenten, Geräten und sonstige Einrichtungen, besonders bevorzugt zur Behandlung medizintechnischer Einmalartikel, insbesondere im Rahmen von Sterilisationsprozessen, mit einem Behandlungsvolumen oder Behandlungsraum, der durch ein unteres Wandelement oder einen Boden, eine Mehrzahl von seitlichen Wandelementen und ein oberes Wandelement oder Decke umgeben ist, wobei der Behandlungsraum zur Erzeugung eines gewünschten Innendrucks darin hermetisch dicht oder abdichtbar und zur Erzeugung einer gewünschten Innentemperatur darin temperierbar ist.

Bei der Herstellung von Artikeln, wie bspw. medizinischen Sterilartikeln, insbesondere von medizinischen Einmalsterilartikeln, werden unterschiedliche Kammern zur produktionstechnischen Behandlung der Artikel eingesetzt, unter anderem Sterilisationskammern für die Sterilisation der Artikel oder Trocknungskammern zum Trocknen der Artikel, insbesondere durch eine Kombination aus Wärme und Vakuum. Üblicherweise werden diese Kammern aus Vollmaterialplatten aus Edelstahl geschweißt und mit einem Medium, wie Wasser oder Luft, über einen Mantel beheizt. Durch Einsatz von Unterdruck im Sterilisationsprozess werden die Kammern hohen Belastungen ausgesetzt und sind daher strukturell unterstützt und mit Verstärkungen versehen. Dies macht die gesamte Konstruktion und Herstellung solcher Kammern sehr aufwendig und verursacht einen hohen Materialeinsatz, da Platten oder Bleche von ausreichender Dicke sowie tragende Elemente zur Verstärkung, Stützung und Absicherung erforderlich sind. Produktionsaufwand und Materialeinsatz sind sehr hoch. Auch sind in der Regel die Lieferzeiten lang. Lieferzeiten von bis zu neun bis zwölf Monate sind keine Seltenheit.

Beim Betrieb einer Sterilisationskammer nach dem Stand der Technik sind des Weiteren große Energiemengen erforderlich, um die Kammer auf eine ausreichende Prozesstemperatur aufzuheizen. Durch die massive Bauweise mit strukturellen Verstärkungs- und Stützelementen ist die zu temperierende Masse groß. Des Weiteren ist das Gewicht der Kammer in nachteiliger Weise in der Regel sehr hoch (eine Kammer mit einer Kapazität für 20 Paletten wiegt ca. 20 t), was eine hohe Bodenbelastung für ein Gebäude darstellt, in dem sich die Kammer befindet, und mit hohen Anforderungen an die Gebäudestatik verbunden ist.

Insgesamt nachteilig bei bekannten Kammern sind ein hoher Materialeinsatz, ein großer Energieaufwand, ein hohes Gewicht, ein hoher Energieverbrauch, ein hoher manueller Arbeitsaufwand sowie lange Lieferzeiten.

Ausgehend davon liegt der vorliegenden Erfindung die Aufgabe zugrunde, eine Kammer zur Produktion von Medizinprodukten, insbesondere von Einmalmedizinprodukten, zu schaffen, die diese Nachteile nicht aufweist. Die Kammer soll insbesondere leicht sein und im Betrieb wenig Energie, insbesondere weniger Energie als vergleichbare bekannte Anlagen, benötigen. Es ist wünschenswert, wenn die Kammer hinsichtlich ihrer Abmessungen ohne großen Aufwand flexibel an am jeweiligen Produktionsstandort vorliegende Gegebenheiten angepasst produziert werden kann. Sie soll des Weiteren einfach mit üblichen Fertigungsmitteln hergestellt werden können.

Diese Aufgabe wird nach der vorliegenden Erfindung gelöst durch eine, insbesondere medizintechnische, Behandlungskammer oder Prozesskammer, insbesondere eine Sterilisationskammer oder eine Desorptionskammer, nach dem Oberbegriff von Anspruch 1, die dadurch gekennzeichnet ist, dass zumindest eines der Wandelemente aus zumindest einem Hohlstrukturprofil oder Hohlkammerprofil ausgebildet ist.

Im Rahmen der vorliegenden Beschreibung der Erfindung ist unter einem Wandelement jedes das Behandlungsvolumen oder den Behandlungsraum begrenzende Bauelement, unabhängig von seiner jeweiligen Orientierung oder Positionierung, zu verstehen. Ein solches Wandelement kann neben Seitenwänden des Weiteren einen Boden, eine Decke, einen Deckel oder eine Tür oder andere Verschlusseinrichtung ausbilden.

Durch den erfindungsgemäßen Einsatz eines Hohlkammerprofils kann die Behandlungs- oder Prozesskammer in besonders vorteilhafter Weise in Leichtbauweise hergestellt werden. Durch die Materialeinsparung bei den Hohlkammerprofilen sind gegenüber herkömmlichen Prozesskammern solche nach der Erfindung bei gleicher Kapazität infolge des geringen Gewichts der Hohlkammerprofile deutlich leichter, so dass statische Anforderungen an Bauwerke, in denen die Kammern aufgestellt sind, geringer sind. Auf der anderen Seite bieten Hohlkammerprofile infolge ihrer inneren Struktur bzw. fachwerkartigen Aufbaus hervorragende statische und dynamische Eigenschaften, wie z.B. Biegesteifigkeit, Verwindungsstabilität oder Lasttragevermögen.

Die im Rahmen der Erfindung genutzten Hohlkammerprofile besitzen in vorteilhafter Weise einen einfachen Aufbau, ein geringes Gewicht und sind zum Beispiel als Strangpressprofile mit zahlreichen unterschiedlichen Profilgeometrien einfach und günstig herstellbar. Der erforderliche Materialeinsatz zum Aufbau eines solchen Hohlkammerprofils und damit eines daraus gebildeten Wandelements ist gering. Insbesondere ist die Masse des Profils und des Wandelements gering, was nicht nur hinsichtlich des Gewichts vorteilhaft ist. Ein aus Hohlkammerprofilen bestehendes Wandelement und damit eine aus solchen Wandelementen bestehende Prozesskammer erfordert infolge der geringen Masse verhältnismäßig geringe Energieströme zum Aufwärmen und zum Abkühlen und ermöglichen daher ein schnelleres Aufheizen und Abkühlen und eine gleichmäßigere Wärmeverteilung als dies bei bekannten Prozesskammern der Fall ist.

Ein besonderer Vorteil ist, dass die Wandelemente wie auch die daraus bestehende Prozesskammer in Modulbauweise konzipiert und erstellt werden kann. Mehrere Hohlkammerprofile können zum Ausbilden eines Wandelements kombiniert und zusammengesetzt werden. Je nach Anzahl der dazu verwendeten Hohlkammerprofile können in einfacher Weise Wandelemente unterschiedlicher Abmessungen hergestellt werden. Durch Verwendung von Hohlkammerprofilen entsprechender Länge kann die Länge der damit zusammengesetzten Wandelemente bestimmt werden. Die Modulbauweise der erfindungsgemäßen Kammer ermöglicht eine schnelle Montage, eine Nutzung von vorgefertigten Normteilen, eine kurze Herstellzeit mit einer somit geringen Lieferzeit, einen einfacher Transport und einen geringen Energieeinsatz.

Hohlkammerprofile besitzen trotz ihres geringen Gewichts eine hohe Stabilität, die konstruktiv gezielt über die Geometrie des Profils, insbesondere über seine Geometrie im Querschnitt bestimmt und gezielt designed werden kann. Insbesondere in Querrichtung kann das Hohlkammerprofil hohe Lasten aufnehmen, ohne sich dabei unerwünscht zu verformen. Ein aus Hohlkammerprofilen bestehendes Wandelement ist damit den im Rahmen von Sterilisationsprozessen, Desorptionsprozessen und insbesondere Unterdruck- oder Vakuumprozessen vorliegenden Druckanforderungen gewachsen.

Vorteilhafte Ausführungsformen der Erfindung sind in den Unteransprüchen beansprucht und werden nachfolgend näher erläutert.

Eine Ausführungsform der Erfindung ist dadurch gekennzeichnet, dass das Hohlkammerprofil ein Strangpressprofil ist. Mit besonderem Vorteil handelt es sich um ein Aluminiumstrangpressprofil. Die Verwendung von Aluminium als Material ist durch den geringeren Materialeinsatz und die hohe strukturelle Festigkeit möglich. Aluminium hat als Material gegenüber Edelstahl, der bislang für solche Prozesskammern eingesetzt wird, deutliche physikalische Vorteile. Zu nennen sind in diesem Zusammenhang unter anderem eine hohe Wärmekapizität, eine gute Wärmeleitfähigkeit sowie ein geringes Gewicht.

Eine weitere Ausführungsform der Erfindung ist dadurch gekennzeichnet, dass das Hohlkammerprofil eine Innenstruktur aus Querstreben oder eine innere Wabenstruktur aufweist. Die Querstreben oder Wandungen der Waben bilden eine Art Stützstruktur aus, die bei geringem Gewicht für eine hohe Stabilität und Steifigkeit des Profils sorgt. Zwischen den Streben und/oder Wandungen können in dem Hohlkammerprofil durchgehende Kanäle ausgebildet sein, die strömungstechnisch zu einem Leitungssystem für ein Temperierungsmedium, ein Behandlungsmedium oder ein Klimatisierungsmedium verbunden sind. Man kann also sagen, dass die Wabenstruktur zusätzlich als Heizkanal oder Temperierungskanal für die Wände genutzt werden kann, was bei bekannten Kammern aus Edelstahl mit Vollmaterialwänden nicht der Fall ist.

Nach einer Ausführungsform der Erfindung kann in dem Hohlkammerprofil ein durchgehender Kanal oder können mehrere durchgehende Kanäle ausgebildet sein. Dieser kann bzw. die können mit Kanälen angrenzender Hohlprofile oder anderen Kanälen des Hohlprofils strömungstechnisch zu einem Leitungssystem für ein Temperierungsmedium, ein Behandlungsmedium oder ein Klimatisierungsmedium verbunden sein. Durchgehend in diesem Zusammenhang bedeutet, dass der Kanal einen Strömungsweg durch das Hohlkammerprofil von einem Eingang zu einem Ausgang bildet.

Nach der Erfindung können mehrere Hohlkammerprofile formschlüssig und/oder kraftschlüssig miteinander verbunden oder verbindbar sein. Es ist besonders vorteilhaft, wenn das Hohlkammerprofil eine Verbindungsstruktur, insbesondere nach Art einer Nut-und-Feder-Verbindung aufweist. Über diese kann das Hohlprofil mit anderen Bauelementen oder mit einem benachbarten Hohlprofil des gleichen Wandelements oder eines angrenzenden Wandelements mediumsdicht miteinander verbunden sein. Die Verbindungsstruktur ist vorzugsweise an einer Stirnseite des Hohlprofils ausgebildet. Vorzugsweise ist auf der jeweils gegenüberliegenden Stirnseite des Hohlprofils ein zur Verbindungsstruktur passender Gegenpart ausgebildet. Die Verbindungsstruktur und ihr Gegenpart können eine Raststruktur aufweisen, die für einen stabilen Zusammenhalt von damit zusammengefügten Profilen sorgt, zumindest, solange diese noch nicht miteinander verschweißt oder anderweitig langfristig verbunden sind.

Eckverbindungen aneinander angrenzender Wandelemente können nach der Erfindung dadurch bewirkt sein, dass randseitig eines Wandelements ein als Eckprofil ausgebildetes Hohlkammerprofil angeordnet ist. Dieses weist einen Knick oder Winkel auf, vorzugsweise einen 90°-Knick, so dass die einander gegenüberliegenden Verbindungsstrukturen ebenfalls um einen Winkel von 90° gegeneinander versetzt sind.

Insbesondere kann die Verbindungstruktur einen Schiebesitz als Toleranzausgleich für eine Schweißmontage ausbilden. Dieser Schiebesitz kann nach der Erfindung zum Beispiel dadurch ausgebildet sein, dass zusammenwirkende Verbindungsstrukturen sowie deren ggf. vorhandene Raststrukturen mit Einlaufschrägen und Auslaufschrägen versehen sind und/oder über eine gewisse Federelastizität verfügen und somit in einem gewissen Rahmen elastisch verformbar sind, ohne dass sich die miteinander in Eingriff stehenden Verbindungsstrukturen voneinander lösen. Man kann auch sagen, dass die Verbindungsstrukturen infolge ihrer Elastizität ein gewisses Spiel von damit zusammengefügten Hohlkammerprofilen ermöglichen.

Mehrere Hohlkammerprofile in horizontaler bzw. vertikaler Ausrichtung können nach der Erfindung stoffschlüssig miteinander verbunden sein. Eine solche stoffschlüssige Verbindung kann alternativ oder zusätzlich zu einer formschlüssigen und/oder kraftschlüssigen Verbindung erfolgen. Um eine stabile und insbesondere mediumsdichte Prozesskammer zu erhalten, sind die Hohlstrukturprofile vorzugsweise miteinander verschweißt. Weitere stoffschlüssige Verbindungen sind möglich und im Bereich der Erfindung, wie zum Beispiel Verkleben oder Verlöten. Ferner können Hohlkammerprofile miteinander verschraubt sein. Für eine weiter vereinfachte Montage können vorgefertigte Bauelemente aus mehreren bereits miteinander verbundenen Hohlkammerprofilen zu Wandelementen verbunden werden.

Durch die Erfindung können insbesondere die folgenden Vorteile erzielt werden: Aufgrund des modularen Aufbaus können verschiedene Kammergrößen schnell gefertigt werden. Profile in unterschiedlichen Höhen und Breiten sind einfach herstellbar und kombinierbar. Die Profile lassen sich einfach auf Länge schneiden. Anzahl und Länge der einzelnen Profile ergeben verschiedene Kammergrößen. Insgesamt wird eine Modulbauweise ermöglicht.

Zusammenfassend kann man auch sagen, dass die Erfindung Prozesskammern, insbesondere Sterilisations- und Desorptionskammern, ermöglicht, die durch Nutzung einer Wabenkonstruktion ein geringes Eigengewicht haben. Durch eine modulare Bauweise sind die Herstellung und der Aufbau einer erfindungsgemäßen Kammer deutlich einfacher und somit kostengünstiger. Strukturelle Anforderungen aufgrund von prozesstechnischen Druckunterschieden können durch die Wabenstruktur besser als bei Kammern nach dem Stand der Technik aufgenommen werden. Die Erfindung ermöglicht insbesondere den Bau einer Sterilisations- oder Desorptionskammer mit Hilfe von Aluminiumstrangpressprofilen. Durch einen wabenförmigen Aufbau der Profile erreicht man eine maximale Stabilität mit geringstmöglichen Gewicht. Des Weiteren können Hohlräume der Struktur oder Profile zum Beheizen oder Kühlen oder Klimatisieren der Kammer verwendet werden. Unter Berücksichtigung der Statik können Kammern unterschiedlicher Größen schnell umgesetzt werden.

Weitere Merkmale und Vorteile der vorliegenden Erfindung ergeben sich aus der folgenden beispielhaften und nicht beschränkenden Beschreibung der Erfindung anhand von Figuren. Diese sind lediglich schematischer Natur und dienen nur dem Verständnis der Erfindung. Dabei zeigen:
Fig. 1 eine perspektivische Darstellung einer Prozesskammer nach der Erfindung ohne stirnseitiges Wandelement;
Fig. 2 eine Schnittansicht der Prozesskammer der Figur 1 entlang der Linie 11-11,
Fig. 3 eine vergrößerte Ansicht eines Eckbereichs der Schnittansicht der Figur 2;
Fig. 4 eine vergrößerte Ansicht des Verbindungsbereichs dreier benachbarter Hohlkammerprofile; und
Fig. 5 eine perspektivische Darstellung vorgefertigter miteinander verbundener Bauelemente.

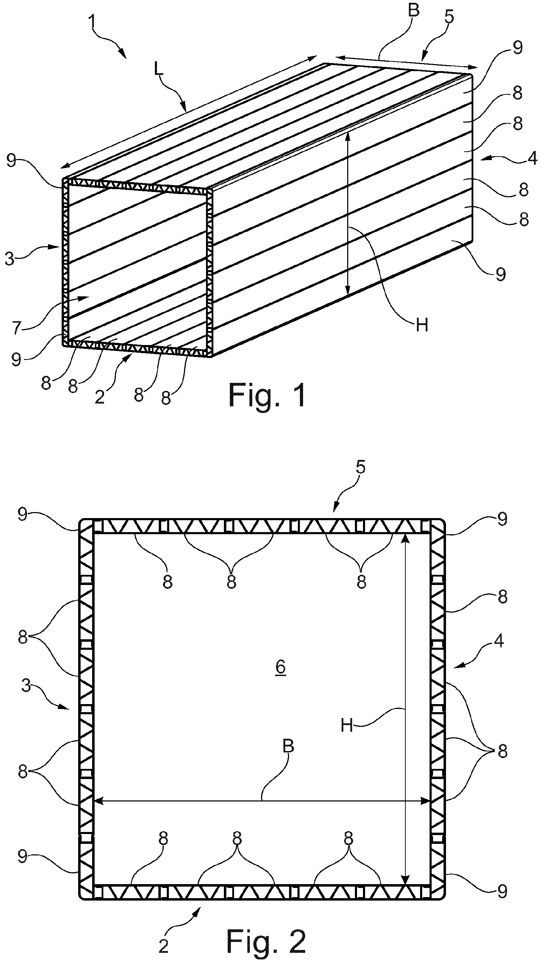

Figur 1 zeigt eine Prozesskammer 1 nach der Erfindung in einer perspektivischen schematischen Ansicht. Die Kammer 1 weist in Figur 1 dargestellt und erkennbar eine Bodenwand 2, zwei Seitenwände 3, 4 und einer Deckenwand 5 auf. Eine in der Figur hintere Stirnwand 6 ist infolge der perspektivischen Darstellung in Figur 1 nicht zu erkennen, in Figur 2 aber angedeutet. Eine in der Figur vordere Stirnwand oder Tür, die die zwischen den Wandelementen 2, 3, 4, 5 liegende Stirnöffnung 7 verschließt, ist aus Gründen der Übersichtlichkeit nicht dargestellt. Jede der vorgenannten sechs Wände bildet ein Wandelement im Sinne der Erfindung aus.

Erfindungsgemäß sind die Wandelemente 2, 3, 4, 5 jeweils als Leichtbauelemente aus einer Mehrzahl von Hohlkammerprofilen 8, 9 zusammengesetzt. Jedes der Hohlkammerprofile 8, 9 weist eine Innenwand 10 und eine dieser gegenüberliegende Außenwand 11 auf. Zwischen der Innenwand 10 und der Außenwand 11 sind Querstreben oder Profilstreben 12 angeordnet. Da das Hohlkammerprofil 8, 9 im vorliegenden Ausführungsbeispiel ein Aluminiumstrangpressprofil ist, sind die Profilstreben 12 jeweils stoffschlüssig sowohl mit der Innenwand 10 als auch mit der Außenwand 11 ausgebildet. Die Profilstreben 12 erstrecken sich in Längsrichtung L des Hohlkammerprofils 8, 9 durchgehend und sind nach Art eines Zick-zack-Musters (gesehen im Querschnitt) angeordnet.

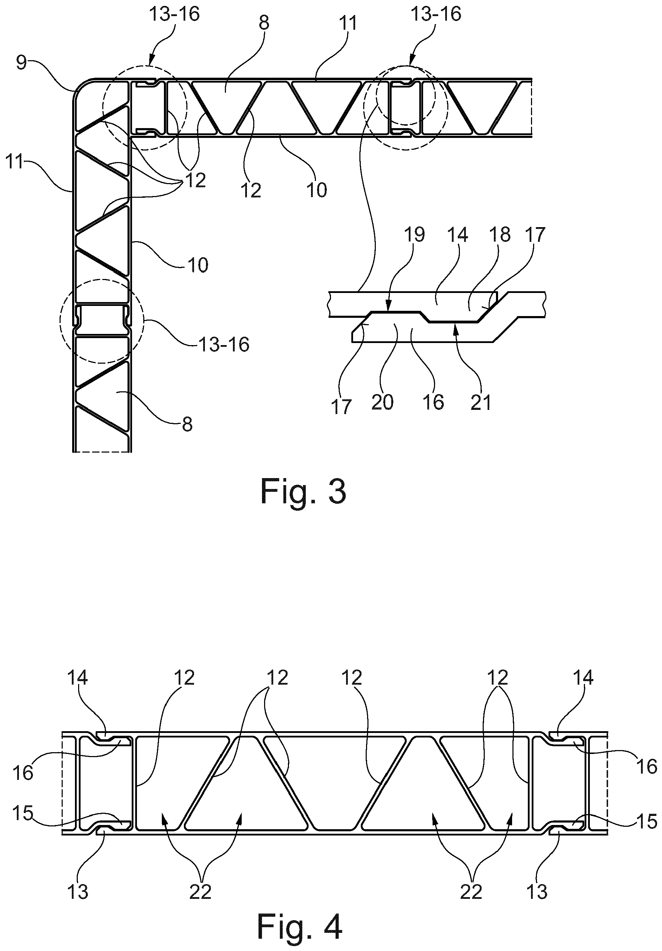

Insbesondere in Figur 3 ist deutlich erkennbar dargestellt, dass es sich bei den Hohlkammerprofilen 8, 9 einerseits um Wandhohlkammerprofile 8 und andererseits um Eckhohlkammerprofile 9 handelt.

Die Wandhohlkammerprofile 8 weisen stirnseitig zwischen den Endabschnitten der Innenwand 10 und der Außenwand 11 Verbindungsstrukturen 13, 14, 15, 16 auf. Einerseits (siehe Figur 4) sind ein Innennutelement 13 und ein Außennutelement 14 ausgebildet. Andererseits, also auf der gegenüberliegenden Seite, sind ein Innenfederelement 15 und ein Außenfederelement 16 ausgebildet. Alle dieser genannten Verbindungsstrukturen weisen einlaufseitig eine Einlaufschräge 17 auf, die ein Zusammenfügen aneinander angrenzender Hohlkammerelemente 8, 9 erleichtern soll. Das Innennutelement 13 und das Außennutelement 14 weisen endseitig jeweils einen nach innen weisenden Nutrastkopf 18 sowie eine sich daran anschließende Nutrastausnehmung 19 auf. Das Innenfederelement 15 und das Außenfederelement 16 weisen endseitig jeweils einen nach außen weisenden Federrastkopf 20 sowie eine sich daran anschließende Federrastausnehmung 21 auf. Wie in Figur 4 dargestellt ist, greift der Nutrastkopf 18 formschlüssig in die Federrastausnehmung 21 ein, während der Federrastkopf 20 formschlüssig in die Nutrastausnehmung 19 eingreift. Die Eckhohlkammerprofile 9 sind ähnlich aufgebaut, mit dem Unterschied, dass die Nutelemente 13, 14 nicht stirnseitig, sondern an der Innenwand 10 angeordnet sind. Selbstverständlich können anstelle der Nutelemente 13, 14 die Federelemente 15, 16 an der Innenwand 10 angeordnet sein.

Die Seitenflächen der Nutrastausnehmung 19 sowie der Federrastausnehmung 21 (und damit auch die Seitenflächen von Nutrastkopf 18 und Federrastkopf 20) sind jeweils geneigt, im vorliegenden Beispiel jeweils um einen Winkel von 45°. Alle Verbindungsstrukturen 13, 14, 15, 16 sind als federelastische Arme ausgebildet, die infolge ihrer Elastizität entweder nach innen (Federrastelemente) oder nach außen (Nutrastelemente) federn. Infolge ihrer Elastizität und der geneigten Seitenflächen ist bei zusammengefügten Hohlkammerprofilen 8, 9 durch die Verbindungsstrukturen 13, 14, 15, 16 eine Art Schiebesitz ausgebildet, der Toleranzen bei einer Schweißmontage ausgleicht.

Zwischen den Querstreben 12, der Innenwand 10 und der Außenwand 11 sind Hohlräume 22 (oder Waben oder Kanäle) ausgebildet. Diese können alle oder zum Teil als Kanäle für ein Temperierungsmedium, ein Behandlungsmedium oder ein Klimatisierungsmedium ausgebildet sein und ein Leitungssystem dafür bilden, mit dem das jeweilige Medium durch die Wandelemente 2, 3, 4, 5, 6 geleitet wird. Diese sind so in einfacher und effektiver Weise temperierbar, insbesondere heizbar und/oder kühlbar, so dass der Innenraum der Prozesskammer 1 temperierbar oder klimatisierbar oder - im Falle von einigen mit dem Innenraum strömungstechnisch verbundenen Kanälen - mit einem bestimmten Schutzgas beaufschlagbar ist.

Wie besonders gut Figur 1 zu entnehmen ist, kann die Länge L der Kammer 1 durch geeignete Ablängung der Hohlkammerprofile bestimmt werden. Wie aus Figur 2 zu entnehmen ist, können die Höhe H und die Breite B der Kammer 1 durch Variation der Anzahl der verwendeten Hohlkammerprofile 8, 9 variiert werden.

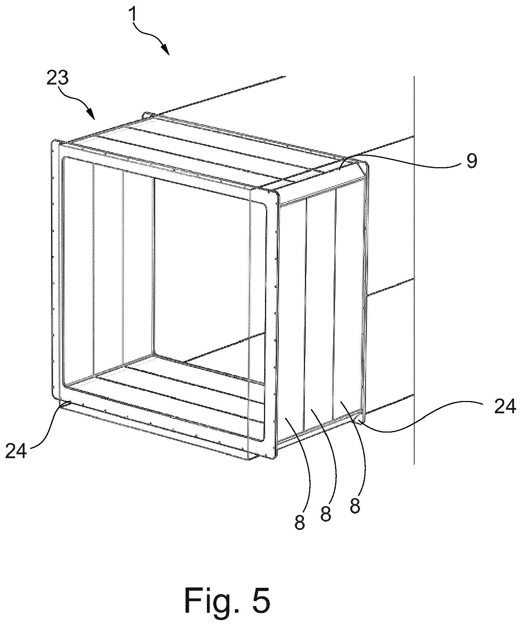

Zum Zwecke einer vereinfachten Montage können mehrere Hohlkammerprofile 8, 9 bereits werksseitig zu vorgefertigten Wandelementen 23 verbunden werden, um die Anzahl der vor Ort zur Montage vorliegenden Elemente zu reduzieren. Figur 5 zeigt eine perspektivische Ansicht solcher miteinander verbundener, vorgefertigter Bauelemente.

Im gezeigten Beispiel sind mehrere Hohlkammerprofile 8, 9 zu einem rechteckigen Hohlprofil verbunden worden. Mehrere als rechteckige Hohlprofile ausgebildete, vorgefertigte Wandelemente 23 können dann hintereinander angeordnet und verbunden werden, um die Kammer 1 zu bilden. Es sind anstelle rechteckiger Hohlprofile auch flächige vorgefertigte Wandelemente 23 oder jede denkbare Kombination von flächigen Wandelementen mit Eckhohlkammerprofilen 9 (z.B. U- oder L-Profile) als vorgefertigte Wandelemente 23 denkbar. Zum Verbinden der vorgefertigten Wandelemente 23 oder zum Anbringen einer vorderen Stirnwand oder Tür können an den Stirnseiten der vorgefertigten Wandelementen Flansche 24 vorgesehen sein.

### Bezugszeichen liste

- 1: Prozesskammer, Behandlungskammer, Kammer
- 2: Bodenwand, Wandelement
- 3: Seitenwand, Wandelement
- 4: Seitenwand, Wandelement
- 5: Deckenwand, Wandelement
- 6: Stirnwand, Wandelement
- 7: Öffnung
- 8: Hohlkammerprofil, Wandhohlkammerprofil
- 9: Hohlkammerprofil, Eckhohlkammerprofil
- 10: Innenwand
- 11: Außenwand
- 12: Profilstreben, Querstreben
- 13: Innennutelement
- 14: Außennutelement
- 15: Innenfederelement
- 16: Außenfederelement
- 17: Einlaufschräge
- 18: Nutrastkopf
- 19: Nutrastausnehmung
- 20: Federrastkopf
- 21: Federrastausnehmung
- 22: Hohlraum
- 23: vorgefertigtes Wandelement
- 24: Flansch
- L: Länge
- B: Breite
- H: Höhe

## Patentansprüche

1. Behandlungskammer (1), insbesondere medizintechnische Behandlungskammer, zur Behandlung von Artikeln, insbesondere von medizintechnischen, Artikeln, Geräten und Einrichtungen, insbesondere im Rahmen von Sterilisationsprozessen, mit einem Behandlungsraum, der durch ein unteres Wandelement (2), eine Mehrzahl von seitlichen Wandelementen (3, 4, 6) und ein oberes Wandelement (5) umgeben ist, wobei der Behandlungsraum zur Erzeugung eines gewünschten Innendrucks darin hermetisch dicht oder abdichtbar und zur Erzeugung einer gewünschten Innentemperatur darin temperierbar ist,
**dadurch gekennzeichnet, dass**
zumindest eines der Wandelemente (2, 3, 4, 5, 6) aus zumindest einem Hohlkammerprofil (8, 9) ausgebildet ist.

2. Behandlungskammer (1) nach Anspruch 1, **dadurch gekennzeichnet, dass** das Hohlkammerprofil (8, 9) ein Strangpressprofil ist.

3. Behandlungskammer (1) nach Anspruch 1 oder 2, **dadurch gekennzeichnet, dass** das Hohlkammerprofil (8, 9) ein Aluminiumstrangpressprofil ist.

4. Behandlungskammer (1) nach einem der vorstehenden Ansprüche, **dadurch gekennzeichnet, dass** das Hohlkammerprofil (8, 9) eine Innenstruktur aus Querstreben (12) oder Profilstreben (12) aufweist.

5. Behandlungskammer (1) nach einem der vorstehenden Ansprüche, **dadurch gekennzeichnet, dass** in dem Hohlkammerprofil (8, 9) zumindest ein durchgehender Hohlraum (22) oder Kanal (22) ausgebildet ist, wobei mehrere Hohlräume (22) oder Kanäle (22) strömungstechnisch zu einem Leitungssystem für ein Temperierungsmedium, ein Behandlungsmedium oder ein Klimatisierungsmedium verbunden sind.

6. Behandlungskammer (1) nach einem der vorstehenden Ansprüche, **dadurch gekennzeichnet, dass** in dem Hohlkammerprofil (8, 9) zumindest ein durchgehender Kanal (22) oder Hohlraum (22) ausgebildet sind, der mit einem Hohlraum (22) oder einem Kanal (22) eines angrenzenden Hohlkammerprofils (8, 9) strömungstechnisch zu einem Leitungssystem für ein Temperierungsmedium, ein Behandlungsmedium oder ein Klimatisierungsmedium verbunden ist.

7. Behandlungskammer (1) nach einem der vorstehenden Ansprüche, **dadurch gekennzeichnet, dass** das Hohlkammerprofil (8, 9) eine Verbindungsstruktur (13, 14, 15, 16), insbesondere nach Art einer Nut-und-Feder-Verbindung, aufweist, und benachbarte Hohlkammerprofile (8, 9) darüber mediumsdicht miteinander verbunden sind.

8. Behandlungskammer (1) nach Anspruch 7, **dadurch gekennzeichnet, dass** die Verbindungstruktur (13, 14, 15, 16) einen Schiebesitz als Toleranzausgleich für eine Schweißmontage ausbildet.

9. Behandlungskammer (1) nach Anspruch 8, **dadurch gekennzeichnet, dass** diese eine Mehrzahl an Hohlkammerprofilen (8, 9) aufweist, die miteinander verschweißt sind.

10. Behandlungskammer (1) nach einem der vorstehenden Ansprüche, **dadurch gekennzeichnet, dass** mehrere miteinander verbundene Hohlkammerprofile (8, 9) ein vorgefertigtes Bauelement (23) bilden.
